Europäisches Patentamt

European Patent Office

Office européen des brevets

(19)

(11) Veröffentlichungsnummer : **0 180 133**
**B1**

(12) ## EUROPÄISCHE PATENTSCHRIFT

(45) Veröffentlichungstag der Patentschrift :
07.06.89

(51) Int. Cl.⁴ : **C 07 C 39/17, C 07 C 37/20**

(21) Anmeldenummer : 85113418.9

(22) Anmeldetag : 22.10.85

(54) Verfahren zur Herstellung von 9,9-Bis(4-hydroxyphenyl)fluoren.

(30) Priorität : 27.10.84 DE 3439484

(43) Veröffentlichungstag der Anmeldung :
07.05.86 Patentblatt 86/19

(45) Bekanntmachung des Hinweises auf die Patenter-
teilung : 07.06.89 Patentblatt 89/23

(84) Benannte Vertragsstaaten :
AT DE FR GB IT NL

(56) Entgegenhaltungen :
EP–A– 0 065 060
DE–A– 2 948 222
DE–B– 1 035 154
Archiv der Pharmazie, 288 (1955), S. 234-246

(73) Patentinhaber : Röhm GmbH
Kirschenallee Postfach 4242
D-6100 Darmstadt 1 (DE)

(72) Erfinder : Riemann, Achim, Dr.
Gisselbergerstrasse 2
D-3550 Marburg (DE)
Erfinder : Ude, Werner, Dr.
Birngartenweg 115
D-6100 Darmstadt (DE)

**Beschreibung**

Gebiet der Erfindung

Die Erfindung betrifft ein Verfahren zur Herstellung von 9,9-Bis-(4-hydroxyphenyl)-fluoren durch Umsetzung von Fluorenon mit Phenol in Gegenwart saurer Kondensationsmittel.

Stand der Technik

Zur Herstellung oben genannter Verbindung, die als Zwischenprodukt, insbesondere zur Herstellung von Polykondensationsprodukten Interesse findet, sind mehrere Verfahren beschrieben, die jedoch gegenüber dem erfindungsgemäßen Vorgehen deutliche Nachteile aufweisen. Diese Kondensationsreaktion ist ein spezieller Fall der Synthese von Dihydroxy-diarylmethan-Derivaten, die durch Kondensation von Carbonylverbindungen mit aromatischen Hydroxyverbindungen in Gegenwart saurer Kondensationsmittel bewerkstelligt wird. Solche bekannte saure Kondensationskatalysatoren sind z. B. Halogenwasserstoffe von Fluor, Chlor oder Brom, andere Halogenverbindungen wie Phosgen, Bortrifluorid, Aluminiumchlorid, weiter Carbonsäuren, Phosphorsäure und auch Schwefelsäure.

Nach dem Stand der Technik kann Schwefelsäure als Kondensationsmittel nur als maximal 70 %ige Säure angewendet werden, da nach H. Schnell und H. Krimm, Angew. Chemie 75 (1963), 663, konzentrierte Schwefelsäure sowohl die aromatischen Hydroxyverbindungen als auch die Dihydroxydiarylmethan-Derivate sulfoniert. Dort wird auch gesagt, daß 70 %ige Schwefelsäure als Kondensationsmittel weniger wirksam ist als konzentrierte Salzsäure und diese wiederum weniger als Chlorwasserstoff.

Für die Herstellung von 9,9-Bis-(4-hydroxyphenyl)-fluoren hat sich, wie aus der US-PS 4 049 721 und aus der DE-OS 29 48 222 hervorgeht, bisher nur Chlorwasserstoff, der gasförmig in das Reaktionsgemisch eingeleitet wird, bewährt. Als effective Cokatalysatoren werden dem Chlorwasserstoff nach der oben genannten US-PS, Beispiel 1, β-Mercaptopropionsäure und nach der oben genannten DE-OS, zwei-, drei- oder vierwertige Metallchloride als zusätzliche Kondensationsmittel hinzugegeben.

Ein Großer Nachteil des Arbeitens mit Chlorwasserstoff oder konzentrierter Salzsäure ist die hohe Korrosivität, die diese Agentien bei großtechnischen Herstellungen von 9,9-Bis-(4-hydroxyphenyl)-fluoren in den entsprechenden Metallapparaturen verursachen. Korrosiv wie Chlorwasserstoff oder konzentrierte Salzsäure wirken auch die als Cokatalysatoren bekannten Metallhalogenide.

Die Korrosivität der HCl-haltigen Reaktionsgemische wird dadurch verstärkt, daß bei der Aufarbeitung noch Wasser zugesetzt wird, und die dann erhaltenen verdünnten HCl-haltigen wäßrigen Lösungen schwierig aufzuarbeiten sind.

Ziel der Erfindung

Der Erfindung liegt die Aufgabe zugrunde, ein Verfahren zur Herstellung von 9,9-Bis-(4-hydroxyphenyl)-fluoren zu schaffen, das das Arbeiten mit chloridhaltigen, korrosiven Reaktionsgemischen vermeidet und ein nicht sulfoniertes Produkt in einer hohen Reinheit und hohen Ausbeute entstehen läßt.

Gegenstand der Erfindung

Überraschenderweise wurde gefunden, daß entgegen der herrschenden Lehrmeinung auch deutlich höher als 70 %ige, d. h. ca. 75 %ige bis 100 %ige Schwefelsäure als Kondensationskatalysator bei der Umsetzung von Fluorenon mit Phenol zu 9,9-Bis-(4-hydroxyphenyl)-fluoren eingesetzt werden kann, ohne daß Sulfonierungsprodukte gebildet werden. Die Umsetzung verläuft schnell zu hoher Ausbeute an reinem Endprodukt und die Korrosivität der chloridhaltigen Reaktionsgemische ist vermieden.

Zur weiteren Erhöhung der Reaktionsgeschwindigkeit werden als Cokatalysatoren Mercaptane, insbesondere Mercaptocarbonsäuren, mit Vorteil β-Mercaptopropionsäure zugesetzt.

Bei Temperaturen über 100 °C werden neben dem gewünschten 9,9-Bis-(4-hydroxyphenyl)-fluoren zunehmend sulfonierte Reaktionsprodukte gebildet. Die dadurch zu erhaltenden niedrigen Ausbeuten an Reaktionsprodukt, das zusätzlich noch durch schwierig abzutrennende Nebenprodukte verunreinigt ist, verbieten ein Arbeiten bei Temperaturen über 100 °C. Die unterste Arbeitstemperaturgrenze von 20 °C, bei der die Reaktion noch mit genügender Geschwindigkeit abläuft, ergibt sich daraus, daß Reaktionsgemische der vorliegenden Erfindung gerade noch in flüssigem Zustand vorliegen. Der Temperaturbereich von 20 bis 70 °C ist bevorzugt.

Ausführung der Erfindung

Die Umsetzung wird zweckmäßig so durchgeführt, daß in geschmolzenes Phenol bei ca. 40-50 °C Fluorenon und β-Mercaptopropionsäure eingerührt werden und in dieses Gemisch die konzentrierte Schwefelsäure so eindosiert wird, daß die Temperatur im Reaktionsgefäß nicht über 100 °C ansteigt. Man setzt das Phenol wegen der Rührbarkeit des Ansatzes vorteilhaft in überstöchiometrischer Menge d. h. bis etwa 6 Mol Phenol pro Mol eingesetztem Fluorenon, ein.

Zur Durchführung der Kondensationsreaktion wird der Kondensationskatalysator Schwefelsäure in Mengen on 0,1 bis 5 Mol, vorzugsweise 0,3 bis 2 Mol $H_2SO_4$ pro Mol Fluorenon eingesetzt. Bevorzugt ist konzentrierte Schwefelsäure (95-100 %ig). Gemäß einer bevorzugten Ausführungsform wird β-Mercaptopropionsäure in Mengen von wenigstens 0,005 Mol pro Mol umzusetzendes Flurenon zugesetzt.

Unter Einhaltung dieser Bedingungen verläuft die Kondensation schnell. Lange Nachreaktionszeiten sind überflüssig, da nach Beendigung der Schwefelsäurezugabe nach etwa einer Stunde ein quantitativer Fluorenonumsatz erzielt ist.

Zur Entfernung von überschüssigem Phenol, von der Schwefelsäure und dem Cokatalysator wird das Reaktionsgemisch zweckmäßigerweise mehrmals mit Wasser, gegebenenfalls unter Mitverwendung eines mit Wasser mischbaren organischen Lösungsmittels, wie z. B. Methanol, ausgekocht, jeweils die wäßrige Lösung abgetrennt und dann das schon praktisch reine Produkt getrocknet. Ein weiteres effectives Verfahren zur Abtrennung des überschüssigen Phenols ist dessen Entfernung durch Wasserdampfdestillation.

Die Erfindung wird durch die folgenden Beispiele näher erläutert.

## Beispiel 1

45 g (0,25 mol) Fluorenon und 94 g (1 mol) Phenol wurden in einem 250 ml-Vierhalskolben, der mit Rührer, Tropftrichter, Thermometer und Rückflußkühler versehen war, auf 30 °C erwärmt und 0,2 ml (2,3 mmol) β-Mercaptopropionsäure hinzugegeben. Anschließend wurde mit Eiswasser gekühlt und 40 ml (0,72 mol) 96 %ige Schwefelsäure so zugetropft, daß die Temperatur im Bereich von 30-70 °C gehalten werden konnte. Die dünnschichtchromatographische Untersuchung des Reaktionsgemisches zeigte, daß nach 15 min der Fluorenonumsatz vollständig war.

Nun wurden 100 ml Methanol hinzugegeben und die Lösung unter Rühren in 1 l kaltes Wasser gegossen, wobei sich unter Entfärbung eine schmierige Masse abschied. Die überstehende wäßrige Methanol/Schwefelsäure-Lösung wurde abgegossen und der Rückstand noch zweimal mit je 1 l Wasser gewaschen. Nach Neutralisation mit wäßriger Ammoniumcarbonatlösung wurde das überschüssige Phenol durch dreimaliges Aufkochen mit je 1 l Wasser entfernt. Das feinkörnige Reaktionsprodukt wurde abgesaugt und bei 60 °C im Vakuumtrockenschrank getrocknet.

Ausbeute : 85,3 g (97,4 % d. Th.)

Das so erhaltene Produkt hatte einen Schmelzpunkt von 222 °C. Durch Umkristallisation aus Isopropylalkohol wurde ein Produkt mit einem Schmelzpunkt von 223 °C (Lit.-Schmelzpunkt : 223°) erhalten.

## Beispiele 2 bis 5

Nach dem in Beispiel 1 beschriebenen Verfahren wurde unter Beibehaltung der Ansatzgröße die Schwefelsäuremenge variiert. Die Ergebnisse sind in Tab. 1 zusammengefaßt.

### Tabelle 1

| Beisp. | Schwefelsäure 96 % | 100 % Umsatz nach | Ausbeute |
|---|---|---|---|
| 2 | 20 ml (0,36 mol) | 0,5 h | 85,0 g (97,0 %) |
| 3 | 10 ml (0,18 mol) | 0,5 h | 84,2 g (96,1 %) |
| 4 | 7,5 ml (0,13 mol) | 0,75 h | 85,5 g (97,6 %) |
| 5 | 5 ml (0,09 mol) | kein vollständiger Umsatz nach 5 h | |

## Beispiel 6

45 g (0,25 mol) Fluorenon und 141 g (1,5 mol) Phenol wurden in einem 500 ml-Vierhalskolben, der mit Rührer, Tropftrichter, Thermometer und Rückflußkühler versehen war, auf 30 °C erwärmt und 0,2 ml β-Mercaptopropionsäure hinzugegeben. Anschließend wurde mit Eiswasser gekühlt und 7.5 ml (0.13 mol) 96 %ige Schwefelsäure so zugetropft, daß die Temperatur im Bereich von 30-70 °C gehalten werden konnte. Nach 45 min wurden 150 ml siedendes Wasser zugegeben, intensiv gerührt und die Emulsion in 0,5 l gerührtes kaltes Wasser gegossen. Die überstehende Lösung wurde dekantiert und der Rückstand zweimal mit je 0,5 l kaltem Wasser gewaschen. Das überschüssige Phenol wurde dann durch fünfmaliges Aufkochen in je 0,5 l Wasser entfernt. Alternativ ließ sich das Phenol auch durch Wasserdampfdestillation abtrennen.

Ausbeute : 85,9 g (98,1 % d. Th.)

Beispiele 7 bis 9

Nach dem in Beispiel 5 beschriebenen Verfahren wurde unter Beibehaltung der Ansatzgröße die β-Mercaptopropionsäuremenge variiert. Die Ergebnisse sind in Tab. 2 zusammengefaßt.

Tabelle 2

| Beisp. | ß–Mercapto-propionsäure | 100 % Umsatz nach | Ausbeute |
|---|---|---|---|
| 7 | 1,0 ml (11,5 mmol) | 0.75 h | 85.5 g (97,6 %) |
| 8 | 0,1 ml ( 1,2 mmol) | kein vollständiger Umsatz nach 5 h | – |
| 9 | ohne ß–Mercapto-propionsäure | kein vollständiger Umsatz nach 5 h | – |

**Patentansprüche**

1. Verfahren zur Herstellung von 9,9-Bis-(4-hydroxyphenyl)-fluoren durch Umsetzung von Fluorenon mit Phenol in Gegenwart saurer Kondensationsmittel als Katalysatoren, dadurch gekennzeichnet, daß als saures Kondensationsmittel Schwefelsäure mit über 70 % Schwefelsäuregehalt eingesetzt wird.

2. Verfahren nach Anspruch 1, dadurch gekennzeichnet, daß konzentrierte (95-100 %ige) Schwefelsäure eingesetzt wird.

3. Verfahren nach den Ansprüchen 1 und 2, dadurch gekennzeichnet, daß die Schwefelsäure in Mengen on 0,1 bis 5 Mol pro Mol umzusetzendes Fluorenon eingesetzt wird.

4. Verfahren nach den Ansprüchen 1 bis 3, dadurch gekennzeichnet, daß die Schwefelsäure in Mengen von 0,3 bis 2 Mol pro Mol umzusetzendes Fluorenon eingesetzt wird.

5. Verfahren nach den Ansprüchen 1 bis 4, dadurch gekennzeichnet, daß als Co-Kondensationsmittel Mercaptane zugesetzt werden.

6. Verfahren nach Anspruch 5, dadurch gekennzeichnet, daß als Mercaptane Mercaptocarbonsäuren verwendet werden.

7. Verfahren nach Anspruch 6, dadurch gekennzeichnet, daß als Mercaptocarbonsäure β-Mercaptopropionsäure eingesetzt wird.

8. Verfahren nach den Ansprüchen 5 bis 7, dadurch gekennzeichnet, daß das Mercaptan in Mengen über 0,005 Mol pro Mol umsuzetzendes Fluorenon eingesetzt wird.

9. Verfahren nach den Ansprüchen 1 bis 8, dadurch gekennzeichnet, daß die Umsetzung im Temperaturbereich von 20 °C bis 100 °C durchgeführt wird.

10. Verfahren nach Anspruch 9, dadurch gekennzeichnet, daß die Umsetzung im Temperaturbereich von 20 bis 70 °C durchgeführt wird.

**Claims**

1. Process for the preparation of 9,9-bis-(4-hydroxyphenyl)-fluorine by reacting fluorinone with phenol in the presence of acid condensing agents as catalysts, characterised in that sulphuric acid having more than 70 % sulphuric acid content is used as an acid condensing agent.

2. Process according to claim 1, characterised in that concentrated (95-100 % strength) sulphuric acid is used.

3. Process according to claims 1 and 2, characterised in that the sulphuric acid is used in amounts of 0.1 to 5 moles per mole of fluorinone to be reacted.

4. Process according to claims 1 to 3, characterised in that the sulphuric acid is used in amounts of 0.3 to 2 moles per mole of fluorinone to be reacted.

5. Process according to claims 1 to 4, characterised in that mercaptans are added as co-condensing agents.

6. Process according to claim 5, characterised in that mercaptocarboxylic acids are used as mercaptans.

7. Process according to claim 6, characterised in that β-mercaptopropionic acid is used as mercaptocarboxylic acid.

8. Process according to claims 5 to 7, characterised in that the mercaptan is used in amounts of more than 0.005 mole per mole of fluorinone to be reacted.

9. Process according to claims 1 to 8, characterised in that the reaction is carried out in the temperature range from 20 ºC to 100 ºC.

10. Process according to claim 9, characterised in that the reaction is carried out in the temperature range from 20 to 70 ºC.


**Revendications**

1. Procédé pour la préparation de 9,9-bis-(4-hydroxyphényl)-fluorène par réaction de la fluorénone sur du phénol en présence d'agents de condensation acides servant de catalyseurs, caractérisé en ce qu'on utilise comme agent de condensation acide de l'acide sulfurique ayant une teneur en acide sulfurique supérieure à 70 %.

2. Procédé selon la revendication 1, caractérisé en ce qu'on utilise un acide sulfurique concentré (à 95-100 %).

3. Procédé selon l'une des revendications 1 et 2, caractérisé en ce que l'acide sulfurique est utilisé en des quantités de 0,1 à 5 moles par mole de fluorénone à faire réagir.

4. Procédé selon les revendications 1 à 3, caractérisé en ce que l'acide sulfurique est utilisé en des quantités de 0,3 à 2 moles par mole de fluorénone à faire réagir.

5. Procédé selon les revendications 1 à 4, caractérisé en ce qu'on ajoute des mercaptans en tant qu'agents de co-condensation.

6. Procédé selon la revendication 5, caractérisé en ce qu'on utilise comme mercaptans des acides mercaptocarboxyliques.

7. Procédé selon la revendication 6, caractérisé en ce qu'on utilise comme acide mercaptocarboxylique l'acide (β-mercaptopropionique).

8. Procédé selon les revendications 5 à 7, caractérisé en ce que le mercaptan est utilisé en des quantités supérieures à 0,005 mole par mole de fluorénone à faire réagir.

9. Procédé selon les revendications 1 à 8, caractérisé en ce que la réaction est mise en œuvre sur l'intervalle de températures de 20 à 100 ºC.

10. Procédé selon la revendication 9, caractérisé en ce que la réaction est mise en œuvre sur l'intervalle de températures de 20 à 70 ºC.